# EUROPEAN PATENT SPECIFICATION

(11) **EP 3 102 942 B1**
(45) Date of publication and mention of the grant of the patent: **12.12.2018**
(21) Application number: 15704264.9
(22) Date of filing: 04.02.2015
(51) Int. Cl.: G01N 33/553

(54) **USE OF RARE METALS AS KEY COMPONENTS**
VERWENDUNG VON SELTENEN METALLEN ALS SCHLÜSSELBESTANDTEILE
UTILISATION DE MÉTAUX RARES COMME COMPOSANTS CLÉS

(30) Priority: 05.02.2014 EP 14153951
(43) Date of publication of application: 14.12.2016
(73) Proprietor: Roche Diabetes Care GmbH, 68305 Mannheim (DE); F. Hoffmann-La Roche AG, 4070 Basel (CH)
(72) Inventor: SCHICK, Wolfgang, 68259 Mannheim (DE); IBACH, Alexander, 77815 Buehl (DE); ISGOEREN, Yilmaz, 67063 Ludwigshafen (DE); KUELLMER, Kai, 61462 Koenigstein im Taunus (DE)
(74) Representative: Herzog, Fiesser & Partner Patentanwälte PartG mbB
(86) International application number: PCT/EP2015/052280
(87) International publication number: WO 2015/118000

(56) References cited:
- EP-A2- 0 821 234
- US-A- 4 752 567
- US-A1- 2003 044 645
- US-A1- 2004 248 318
- US-A1- 2006 147 927
- US-A1- 2010 010 630

## Description

The present invention pertains to the field of manufacture of diagnostic test elements. Specifically, the present invention relates to the use of a rare metal component, in particular rare alkali metal components, comprised in a coating composition for determining the amount of dried coating composition in a coat. Moreover, the invention encompasses a method for determining the amount of dried coating composition in a coat. Further, the invention pertains to a coating composition comprising a rare metal component, in particular a rare alkali metal component, and the use of said coating composition for the manufacture of a diagnostic test element as well as to the said test element.

Coating processes are used in many areas of the art. In particular, the manufacture of diagnostic test elements useful in the measurement of biological analytes such as blood glucose require the generation of different layer films on a solid support. Theses layers are obtained by a coating process wherein one or more coating compositions are applied to the solid support. Typical diagnostic test elements and coating processes for the generation thereof are described in, e.g., DE 196 29 656 A1, DE 196 29 657 A1, WO 2010/052306 or EP 0 821234 B1.

However, it is decisive for reliably working diagnostic test elements that the quality of the coat can be assured. To this end, it is necessary to characterize the coat, i.e. the layers, by determining the amount of dried coating composition which forms the said coat. Such a characterization is required to assess the quality of the coat in the context of desired specifications.

Currently, the amount of dried coating composition applied to a solid support is determining by differential weighing, i.e. by weighing the amount of coating composition prior and after application. Thereby, the amount of coating composition which has been applied can be calculated. Moreover, the amount of dried coating composition is subsequently determined by calculating the sum of the amounts of the solid components of the composition.

Differential weighing may also be used to determine the applied dried coating composition directly. To this end, the solid support with the coat of dried coating composition is weighed first. Afterwards, the dried coat is completely removed from the solid support, e.g., by ultrasound treatment, and the solid support without the coat is weighed again. The determined difference of the weights represents the amount of dried coating composition applied as a coat to the solid support.

The amount of dried coating composition in a coat can also be determined by infrared (IR) spectroscopic techniques or by determination of heavy elements via X-ray fluorescence (for determination of heavy elements by X-ray fluorescence, see Rizescu 2001, International Journal of Energy and Environment 4(5):503-513; Ene 2010, Rom Journal Phys. 55(7-8): 815-820). These techniques are, however, sensitive to matrix effects of the coat or require the addition of high concentrations of heavy elements to the coat. US 4,752,567 discloses a method of microscopic detection of rare metal particles to determine the thickness of a coating.

Thus, there is a need for an improved technique for determining the quality of a coat and, in particular, the applied dried coating composition.

The technical problem underlying the present invention can be regarded as the provision of means and methods for complying with the aforementioned needs. The said technical problem is solved by the embodiments characterized in the claims and herein below.

Accordingly, the present invention relates to the use of a rare metal component comprised in a coating composition for determining the amount of dried coating composition in a coat according to claim 1. Further, the present disclosure relates, in general, to the use of a rare metal component comprised in a coating composition for determining the amount of dried coating composition in a coat.

The term "rare metal component" as used herein refers to a composition comprising a rare metal atom or ion. Rare in this context is to be understood that the metal ion or atom shall not be present in other components of the coating composition in detectable amounts. Accordingly, it shall be a unique component of the coating composition. Therefore, frequently occurring metal ions which are part of salts used for conventional buffers or the like shall not be considered to be rare. Rare metals and metal ions may be selected from the group of heavy metals including transition metals, metalloids, lanthanides and actinides, and some alkali metals.

Particular envisaged as rare heavy metals in accordance with the present invention are those are those having a density of greater 5 g / m³, more typically, greater 10 g / cm³ and most typically, 20 g / m³. Heavy metals having a density of at least 5 g / m³ are V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf or Es. Moreover, it is particularly envisaged that the rare heavy metal atom or ion used in the rare heavy metal component of the invention shall be positively charged after ionization in the context of MS analysis. Moreover, it is envisaged that the rare heavy metal component shall not interfere with other components of the coating composition and their purpose in the coat. For example, for most purposes of a coat, non-radioactive heavy metals may de desired.

In one aspect, the rare metal component is a rare alkali metal component. The term "rare alkali metal component" as used herein refers to a composition comprising a rare alkali metal atom or ion. Frequent alkali metals in accordance with the present invention are sodium (Na) and potassium (K). Accordingly, a rare alkali metal component in accordance with the present invention comprises a lithium (Li), rubidium (Rb), cesium (Cs) or francium (Fr) atom or ion. Composition which may be suitable as rare alkali metal components for the coating composition to be used according to the present invention are salts, hydroxides or organometallic compounds comprising the said rare alkali metal atoms or ions. Particular envisaged are salts, such as halogen salts, and hydroxides of the rare alkali metals. Thus, in an aspect, the rare alkali metal components according to the present invention may be LiCl, RbCl, CsCl or FrCl. Instead of the chloride (Cl-) anion, any other halide may be used, such as fluoride (F-), bromide (Br-), iodide (I-) or astatide (At-). Thus, in another aspect, the rare alkali metal components according to the present invention may be LiF, RbF, CsF or FrF or LiBr, RbBr, CsBr or FrBr or Lil, RbI, CsI or FrI or LiAt, RbAt, CsAt or FrAt. Other salt forming anions can be used as well. In another aspect, the rare alkali metal components according to the present invention may be LiOH, RbOH, CsOH or FrOH. In particular, LiOH or RbOH are suitable rare alkali metal components in accordance with the present invention.

The term "coating composition" as used herein refers to a liquid composition which is capable of forming a coat upon application onto, e.g., a solid support. A "coat" as referred to herein is formed by removing the solvent from the coating composition such that a dry layer of coating composition remains on the solid support as coat. Depending on the desired purpose of the coat, the coating composition may comprise various components. However, it will be understood that the coating composition shall comprise a removable solvent, such as an aqueous solvent. Aqueous solvents, e.g., can be removed from the coating composition by applying heat treatment, evaporation or freeze-drying. Other removable solvents, such as organic solvents, are also conceivable in accordance with the present invention. The coating composition according to the present invention, however, shall comprise at least one rare metal component as specified above. In one aspect, the at least one rare metal component is at least one rare alkali metal component. In an aspect, the said component is comprised in the coating composition in pre-defined amounts such that based on the pre-defined amount of the rare metal components the applied coating composition forming coat can be precisely determined even after removal of the solvent. It will be understood that depending on the application technique used for applying the coating composition to the support, different amounts may be applied. Even if the same technique is used for application, the applied amounts may vary from coated support to coated support. Based on the amount of rare metal component determined in the coat, it is possible to compare the amounts of dried coating composition applied to different supports, e.g., as a measure of quality control for the coating process.

In a particular aspect of the use of the present invention, said coating composition is capable of forming a detection layer or a reaction layer used in a diagnostic test element.

Diagnostic test elements, in principle, comprise at least one detection reagent for the qualitative and/or quantitative detection of an analyte. A detection reagent is to be generally understood to mean a chemical substance or a mixture of chemical substances which, in the presence of the said analyte, changes at least one detectable property, more particularly a physically and/or chemically detectable property. Preferably, this property change occurs specifically only in the presence of the analyte to be detected, but not in the presence of other substances.

The at least one property change can be, for example, the change in an optically detectable property, more particularly a color change. Examples of diagnostic test elements having optical detection reagents are well known in the prior art. For example, DE 196 29 656 A1, DE 196 29 657 A1, WO 2010/052306 or EP 0 821 234 B1 describe diagnostic test elements for determining an analyte from whole blood by means of a reagent system which is present in the element and which includes a color formation reagent. Such a diagnostic test element comprises a test field with a sample loading side, onto which the sample is added, and a detection side, on which an optically detectable change occurs as a result of the reaction of the analyte with the reagent system. The test field is configured such that the erythrocytes present in the blood sample do not reach the detection side. Furthermore, the test field has a transparent slide and a first film layer and also a second film layer applied to the first film layer. The first layer or reaction layer located on the transparent slide is in a moist state and thereby exhibits considerably less light scattering than the second layer lying over it. It comprises the detection reagents for detecting the analyte. The first film layer comprises a filler whose refractive index is close to the refractive index of water, whereas the second layer or detection layer contains a pigment having a refractive index of at least or even > 2.0, more particularly of at least 2.2, at a concentration of at least 25% by weight or even more than 25% by weight, based on the dried second layer. For example, the first layer can comprise a sodium aluminum silicate as filler.

The term "reaction layer" (or "first layer") as used herein, thus, refers to a film layer in the test field which comprises the reaction reagent(s) for detecting the analyte. Moreover, the said layer shall also comprise coating compounds which contain polymeric film formers, swelling agents and weakly light scattering fillers or no fillers at all. Weakly light fillers are those in accordance with the present invention whose refractive index is near to the refractive index of water. Silicone dioxide, silicates and aluminum silicates have proven to be particularly suitable for this purpose.

The term "detection layer" (or "second layer") as used herein refers to a film layer in the test field which comprises dispersion-saturated solid components. Typically, the detection layer requires a swelling agent and in any case at least one pigment scattering light strongly. Ideally the refractive index of the pigments in the second film layer should be at least 2.5. Hence TiO₂ is typically added to the layer. In addition the second layer can also contain non-porous fillers as well as porous fillers. By adding a swelling agent that swells well (i.e. a substance which increases its volume when it takes up water) one does not only obtain layers which can be penetrated relatively rapidly by sample liquid but have good erythrocyte and additionally also blood pigment separation properties despite this opening effect of the swelling agent. The swelling properties should be so good that for a test in which the rate of color formation--such as for example of a glucose test reaction--is mainly dependent on the penetration of the sample liquid through the layer, the optically detectable reaction is measurable after a maximum of one minute. Especially suitable swelling agents have proven to be methyl vinyl ether maleic acid anhydride copolymer, xanthan gum and methyl vinyl ether maleic acid copolymer. It will be understood that one or more reaction layers may be used in accordance with the diagnostic test element of the present invention.

The coating composition which is capable of forming the detection layer or reaction layer is typically an aqueous composition. The components of the said detection layer or reaction layer are also contained in the said coating composition. Moreover, the said composition will comprise suitable solvents for said components well known to the skilled artisan from, e.g., DE 196 29 657 A1, DE 196 29 656 A1, EP 0 821 234 B1, EP 1 035 919 B1 or EP 1 035 920 B1.

The manufacture of diagnostic test elements comprising test fields having a multiple layer structure is, in principle, well known to the person skilled in the art and described, e.g., in DE 196 29 657 A1, DE 196 29 656 A1 or EP 0 821 234 B1. In an aspect, a coating composition for the reaction layer is applied to the test field on the diagnostic element first. Subsequently, the solvent is removed from the coating composition resulting in the formation of a dry coat being the first layer, i.e. the reaction layer. In a further step, the coating composition for the detection layer is applied to the first layer. The solvent is again removed in order to generate the second layer, i.e. the detection layer. The solvent can be removed from the coating composition after application of the said composition to the test field of the diagnostic test element by all techniques known for removing solvents including heat treatment, evaporation or freeze-drying.

In an aspect, the detection layer of a diagnostic test device is formed from a coating composition comprising a first rare metal component, while the reaction layer is formed from a coating composition comprising a second rare metal component, whereby the first and the second rare metal components differ with respect to the rare metal atom or ion. In such an aspect, it is possible to use the different rare metal components for determining the amount of both, the dried coating composition being the detection layer and the dried coating composition being the reaction layer. In one aspect, the at least one rare metal component in the coating composition is at least one rare alkali metal component. In a particular aspect, RbOH and LiOH are applied as first and second rare alkali metal components.

The term "amount" as used herein refers to an absolute amount or a relative amount, i.e. a concentration of the components in a certain volume. It will be understood that based on the amount of dried coating composition, other parameters characterizing a coat such as its thickness or its weight can be calculated as well.

Advantageously, it has been found in the studies underlying the present invention that rare metal components, such as hydroxides or salts of the said rare metals, can be used as key-components in pre-defined amounts in a coating composition. In one aspect, the rare metal components are rare alkali metal components. Based on these key-components, the amount of dried coating composition in a coat, e.g., a reaction or detection layer in a diagnostic test element, can be precisely determined. Since the key-components serve as detectable labels for the coat, it will be understood that the rare metal atom or ion of the rare metal component shall not be introduced from components other than the said key-component or by contaminations. Rare metal components, in particular rare alkali metal components, are particularly well suited as key-components in detection or reaction layers comprised in diagnostic test elements because these components are rather inert with respect to other chemical reactions which need to be carried out in the said layers. Moreover, they do not significantly alter required physical properties such as optical properties of the layers. A further advantage of using rare metal components, in particular rare alkali metal components, as key-components is that these rare metal atoms or ions comprised in the components can be detected at very low detection levels, e.g., in the ppb (ng/g) range. To this end, mass spectroscopy such as ICP-MS turned out to be a favorable detection technique in accordance with the findings underlying the present invention. Moreover, isotopes of the rare metals can be used as internal standards for calibration purposes, whereby a precise and quantitative determination of the amount of rare metal component in a dried coating composition is allowed. Thanks to the present invention, quality of coatings, in particular in the field of diagnostic test elements, can be significantly improved.

The definitions and explanations of the terms made before apply mutatis mutandis for the embodiments described below.

In the following, particular embodiments of the use of the invention are specified:
In an embodiment of the use of the present invention, said rare metal component is a rare alkali metal component.

In a furthermore embodiment of the use of the present invention, said rare alkali metal component comprises a Lithium, Rubidium or Cesium ion.

In a further embodiment of the use of the present invention, said rare metal component is a rare alkali metal hydroxide or salt.

In yet an embodiment of the use of the present invention, said rare metal component is present in the coating composition in a pre-defined amount.

In an embodiment of the use of the present invention, said rare metal component does not react with other components of the coating composition.

In another embodiment of the use of the present invention, said coat is a detection layer or a reaction layer.

In a further embodiment of the use of the present invention, said detection layer or reaction layer is comprised by a diagnostic test element.

Moreover, the present invention relates to a method for determining the amount of dried coating composition in a coat according to claim 7. Further, the present disclosure relates to a method for determining the amount of dried coating composition in a coat, said method comprising:
a) dissolving the coat comprising dried coating composition in a solvent, wherein said coating composition comprises a rare metal component and wherein said solvent comprises an internal standard for the said rare metal component;
b) detecting the amount of the rare metal component; and
c) determining the amount of dried coating composition comprised in the coat based on the detected amount of the rare metal component.

Dissolving the coat as referred to herein comprises mobilizing the individual components of the dried coating composition in the coat and bringing them into solution. Typically, the coat or a pre-defined part thereof will be dissolved in an acidic aqueous solution such as a HNO₃ solution. In addition, the dissolving may also comprise the application of physical forces, e.g., by shaking or the application of microwave irradiation.

Typically, an internal standard for the rare metal component, in particular a rare alkali metal component, is added to the dissolved coat in a pre-defined amount. Such a standard may comprise or consist of an isotope of the rare metal or may be a compound comprising it. Based on the said standard which is added in known, pre-defined amount to the solution, a calibration for different amounts of the rare metal component is feasible.

The detection of the rare metal component, in particular the rare alkali metal component, can be carried out by various detection techniques which allow for a specific quantitative determination of the rare metal atoms or ions comprised in the solution comprising the dissolved coat. In particular, spectroscopy techniques are suitable for determining the rare metal atoms or ions based on their physical and/or chemical properties. Nevertheless, other techniques are also encompassed according to the present invention. In particular, mass spectroscopy techniques, such as inductive coupled mass spectroscopy (ICP-MS), give particular favorable results when applied in the method according to the present invention. Techniques such as infrared (IR) spectroscopy are dependent on matrix effects or, in the case of, e.g., X-ray fluorescence analysis, require heavy key-components in rather high concentrations to be present in the coating composition.

The amount of dried coating composition comprised in the coat can be determined based on the detected amount of the rare metal component. To this end, the determined amount is compared with the pre-defined amounts of internal standard such that the amount can be calculated quantitatively.

In an aspect, the method encompasses dissolving two different coats, such as a reaction and a detection layer of a diagnostic test element, whereby the coats comprise different rare metal components. Thereby, the method of the present invention allows for determining the amount of coating composition in each of the different coats within one process since the one coating composition is represented by a first metal component while the other coating composition is represented by a second rare metal component differing from the first one with respect to the rare metal atom or ion. Thus, the method of the present invention allows for determining the relative amounts as well as the absolute amounts of the coating compositions in different layers in relation to each other. By comparing the said absolute or relative amounts in two different layers, ratios may be calculated and established for, e.g., quality control purposes in the coating process. In certain aspects, the rare metal components are rare alkali metal components.

In the following, particular embodiments of the diagnostic test element of the invention are specified:
In an embodiment of the method of the present invention, said rare metal component is a rare alkali metal component, in particular, as specified elsewhere herein.

In an embodiment of the method of the present invention, said amount of the rare metal component is detected by mass spectroscopy.

In a further embodiment of the method of the present invention, said mass spectroscopy is inductive coupled mass spectroscopy (ICP-MS).

In yet an embodiment of the method of the present invention, said dissolving comprises acidic treatment and/or microwave treatment.

In an embodiment of the method of the present invention, said internal standard is an isotope enriched rare metal component.

In a further embodiment of the method of the present invention, two coats comprising a first and a second dried coating composition, respectively, are dissolved,
wherein the first coating composition comprises a first rare metal component in a solvent, said solvent comprises an internal standard for the said first rare metal component, and
wherein the second coating composition comprises a second rare metal component in a solvent, said solvent comprises an internal standard for the said second rare metal component.

In a particular embodiment, the said method further comprises comparing the amount of dried coating composition comprised in the said first and the said second coat.

In another embodiment of the method of the present invention, two coats comprising a first and a second dried coating composition, respectively, are dissolved in at least one solvent,
wherein the first coating composition comprises a first rare metal component,
wherein the second coating composition comprises a second rare metal component, and
wherein said at least one solvent comprises at least one internal standard for the said first rare metal component and/or for the said second rare metal component,
thereby determining the absolute and/or relative amount of said first and said second dried coating composition comprised in said two coats based on the detected amount of the said first and the said second rare metal component.

The present invention also contemplates a coating composition for forming a coat being a detection layer or a reaction layer comprised by a diagnostic test element according to claim 13. Further, the present disclosure relates to a coating composition for forming a coat being a detection layer or a reaction layer comprised by a diagnostic test element, wherein said coating composition comprises a rare metal component, in particular a rare alkali metal component.

Further encompassed by the present invention is a diagnostic test element according to claim 14. Said test element shall comprise a coat being a detection layer or a reaction layer, said coat comprising dried coating composition comprising a rare metal component, in particular a rare alkali metal component.

In an aspect, the said diagnostic test element comprises a coat being a detection layer and a coat being a reaction layer, wherein the coat being the detection layer comprises a rare metal component which differs with respect to the metal from the metal component comprised in the coat being the detection layer. Thereby, it is possible to determine the amount of dried coating composition in different coats using the same analysis, e.g., mass spectroscopy such as ICP-MS.

Finally, the invention relates to the use of the coating composition of the invention for the manufacture of a diagnostic test element according to claim 15. Further, the present disclosure relates to the use of the coating composition of the invention for the manufacture of a diagnostic test element comprising said coating composition in a coat being a reaction layer or a reaction layer.

### FIGURES

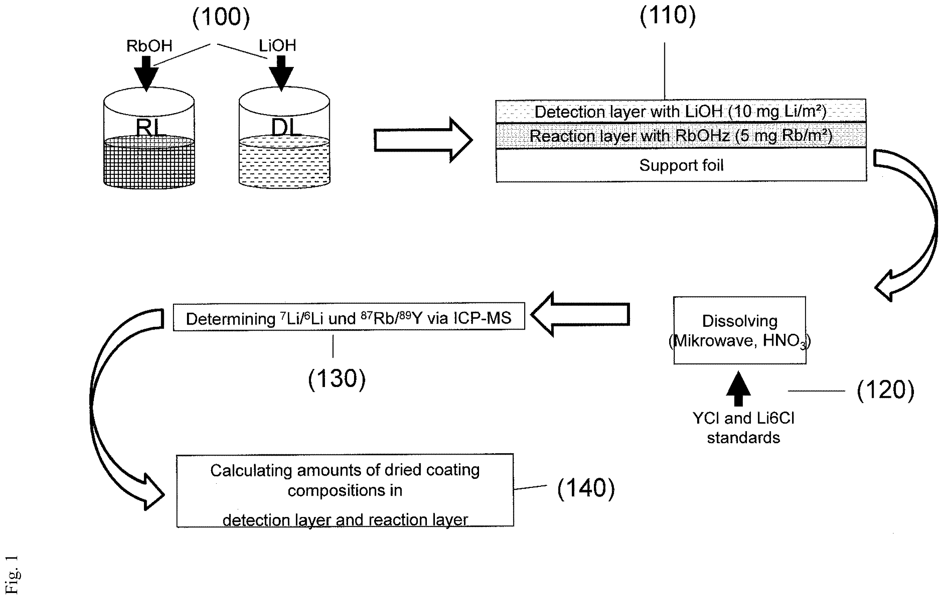

**Fig. 1** shows a schematic drawing of the method of an embodiment of the method of the invention. Coating compositions for the detection and reaction layers are generated with rare alkali metal components as key components (100). An area of a test field (110) is dissolved and an internal standard for the included key component, i.e. the rare alkali metal component, is added (120). Subsequently, the content of rare alkali metal atoms or ions is determined by mass spectroscopy (130), such as ICP-MS. Based on the content of rare alkali metal component, the amount of applied dried coating composition is calculated (140).

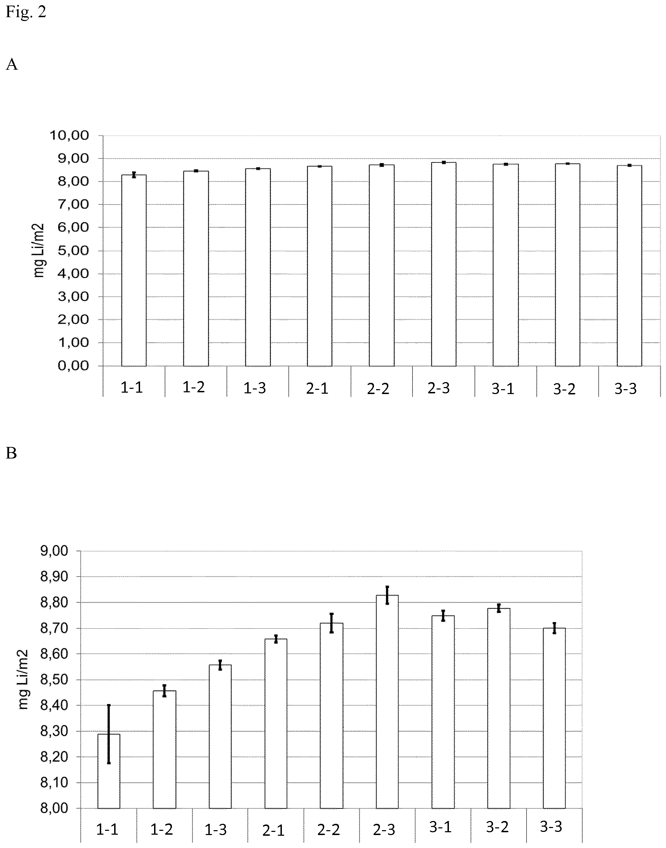

**Fig.2** shows ICP-MS analyses for determining the amount of Lithium ions present in a coat obtained from a LiOH containing coating composition. (A) Analysis of detection layers on different test elements. (B) Scale-up of the diagram of (A) in the range between 8.00 and 9.00 mg/m². Numbering 1-1, 1-2, and 1-3 for the three test pieces of the first pre-cut blank, 2-1, 2-2, 2-3 for the three test pieces of the second pre-cut blank, and 3-1, 3-2, and 3-3 for the three pieces of the third pre-cut blank.

### EXAMPLES

The following Examples shall merely illustrate the invention or aspects thereof. They must, however, not be construed in any way which limits the scope of the invention.

### Example 1: Manufacture of diagnostic test elements with coating compositions comprising rare alkali metals as key components

Coating compositions for detection and reaction layers were generated essentially as described in in DE 196 29 657 A1, DE 196 29 656 A1 or EP 0 821 234 B1.

In addition, however, LiOH was added to the detection layer coating composition in an amount such that the final coat shall comprise 10 mg Li⁺ / m². RbOH was added to the reaction layer coating composition in an amount such that the final coat shall comprise 10 mg Rb⁺ / m². To this end, RbOH was added in a concentration of 0.01 g / 100 g coating composition to the reaction layer coating composition, while LiOH was added in a concentration of 0.06 g / 100 g coating composition to the detection layer coating composition.

### Example 2: Measurement of the precise amounts of dried coating compositions in the coats using ICP-MS

A 1 cm² area of a test field of a diagnostic test element manufactured as indicated in Example 1 was dissolved in HNO₃ and treated in the microwave until the layers and the support matrices were entirely dissolved (110). Y⁸⁹Cl and Li⁶Cl were added in predefined amounts as internal calibration standards to different samples (120). ICP-MS was carried out in order to determine the ratios of Li⁷/Li⁶ and Rb⁸⁶/Y⁸⁷ (130). The amount of dried composition in the detection and reaction layer was calculated (140) based on the amounts used for the generation of the coating compositions and the ratios of the isotopes determined by ICP-MS (see Fig. 1).

The results obtained for different test elements are shown in Fig. 2. In particular, Fig. 2 (B) shows that minor differences in the amount of dried coating composition can be determined based on the Li ions as key components by ICP-MS. Three test pieces of three pre-cut blanks were investigated (numbering 1-1, 1-2, and 1-3 for the three test pieces of the first pre-cut blank, 2-1, 2-2, 2-3 for the three test pieces of the second pre-cut blank, and 3-1, 3-2, and 3-3 for the three pieces of the third pre-cut blank).

## Claims

1. Use of a rare metal component comprised in a coating composition for determining the amount of dried coating composition in a coat, wherein said rare metal component is a composition comprising a rare metal atom or ion selected (i) from V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf and Es, or (ii) from Li, Rb, Cs and Fr,
wherein said coat is a detection layer or a reaction layer
and wherein said determining comprises dissolving said coat in a solvent.

2. The use of claim 1, wherein said rare metal component is a rare alkali metal component, in an embodiment, wherein said rare alkali metal component comprises a Lithium, Rubidium or Cesium ion.

3. The use of claim 1 or 2, wherein said rare metal component is a rare metal hydroxide or salt.

4. The use of any one of claims 1 to 3, wherein said rare metal component is present in the coating composition in a pre-defined amount.

5. The use of any one of claims 1 to 4, wherein said rare metal component does not react with other components of the coating composition.

6. The use of any one of claims 1 to 5, wherein said detection layer or reaction layer is comprised by a diagnostic test element.

7. A method for determining the amount of dried coating composition in at least one coat, said method comprising:
a) dissolving the said at least one coat comprising dried coating composition in a solvent, wherein said coating composition comprises a rare metal component and wherein said solvent comprises an internal standard for the said rare metal component, wherein said rare metal component is a composition comprising a rare metal atom or ion selected (i) from V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf and Es, or (ii) from Li, Rb, Cs and Fr;
b) detecting the amount of the said rare metal component; and
c) determining the amount of dried coating composition comprised in the said at least one coat based on the detected amount of the rare metal component;
wherein said at least one coat is a detection layer or a reaction layer.

8. The method of claim 7, wherein said rare metal component is a rare alkali metal component.

9. The method of claim 7 or 8, wherein said amount of the rare metal component is detected by mass spectroscopy, in an embodiment, wherein said mass spectroscopy is inductive coupled mass spectroscopy (ICP-MS).

10. The method of any one of claims 7 to 9, wherein said dissolving comprises acidic treatment and/or microwave treatment.

11. The method of any one of claims 7 to 10, wherein said internal standard is an isotope enriched rare metal component.

12. The method of any one of claims 7 to 11, wherein two coats comprising a first and a second dried coating composition are dissolved in at least one solvent,
wherein the first coating composition comprises a first rare metal component, wherein the second coating composition comprises a second rare metal component, and
wherein said at least one solvent comprises at least one internal standard for the said first rare metal component and/or for the said second rare metal component,
thereby determining the absolute and/or relative amount of said first and said second dried coating composition comprised in said two coats based on the detected amount of the said first and the said second rare metal component.

13. A coating composition for forming a coat being a detection layer or a reaction layer comprised by a diagnostic test element, wherein said coating composition comprises a rare metal component, wherein said rare metal is a metal not present in other components of the coating composition in detectable amounts, and wherein said rare metal component does not significantly alter the optical properties of the layer.

14. A diagnostic test element comprising a coat being a detection layer or a reaction layer, said coat comprising dried coating composition comprising a rare metal component, wherein said rare metal is a metal not present in other components of the coating composition in detectable amounts, wherein said rare metal component does not significantly alter the optical properties of the layer.

15. Use of the coating composition of claim 13 for the manufacture of a diagnostic test element comprising said coating composition in a coat being a detection layer or a reaction layer.

## Patentansprüche

1. Verwendung eines Seltenmetallbestandteils in einer Beschichtungszusammensetzung zum Bestimmen der Menge an getrockneter Beschichtungszusammensetzung in einer Schicht, wobei es sich bei dem Seltenmetallbestandteil um eine Zusammensetzung handelt, die ein Seltenmetallatom oder -ion umfasst, ausgewählt (i) aus V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf und Es oder (ii) aus Li, Rb, Cs und Fr,
wobei es sich bei der Schicht um eine Detektionsschicht oder eine Reaktionsschicht handelt
und wobei das Bestimmen das Lösen der Schicht in einem Lösungsmittel umfasst.

2. Verwendung nach Anspruch 1, wobei es sich bei dem Seltenmetallbestandteil um einen Seltenalkalimetallbestandteil handelt, wobei in einer Ausführungsform der Seltenalkalimetallbestandteil ein Lithium-, Rubidium- oder Caesiumion umfasst.

3. Verwendung nach Anspruch 1 oder 2, wobei es sich bei dem Seltenmetallbestandteil um ein Seltenmetallhydroxid oder -salz handelt.

4. Verwendung nach einem der Ansprüche 1 bis 3, wobei der Seltenmetallbestandteil in einer vordefinierten Menge in der Beschichtungszusammensetzung vorliegt.

5. Verwendung nach einem der Ansprüche 1 bis 4, wobei der Seltenmetallbestandteil nicht mit anderen Bestandteilen der Beschichtungszusammensetzung reagiert.

6. Verwendung nach einem der Ansprüche 1 bis 5, wobei die Detektionsschicht oder Reaktionsschicht in einem Diagnosetestelement enthalten ist.

7. Verfahren zum Bestimmen der Menge an getrockneter Beschichtungszusammensetzung in mindestens einer Schicht, wobei das Verfahren Folgendes umfasst:
a) Lösen der mindestens einen Schicht, die getrocknete Beschichtungszusammensetzung umfasst, in einem Lösungsmittel, wobei die Beschichtungszusammensetzung einen Seltenmetallbestandteil umfasst und wobei das Lösungsmittel einen internen Standard für den Seltenmetallbestandteil umfasst, wobei es sich bei dem Seltenmetallbestandteil um eine Zusammensetzung handelt, die ein Seltenmetallatom oder -ion umfasst, ausgewählt (i) aus V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf und Es oder (ii) aus Li, Rb, Cs und Fr;
b) Detektieren der Menge des Seltenmetallbestandteils; und
c) Bestimmen der Menge an getrockneter Beschichtungszusammensetzung, die in der mindestens einen Schicht enthalten ist, basierend auf der detektierten Menge des Seltenmetallbestandteils;
wobei es sich bei der mindestens einen Schicht um eine Detektionsschicht oder eine Reaktionsschicht handelt.

8. Verfahren nach Anspruch 7, wobei es sich bei dem Seltenmetallbestandteil um einen Seltenalkalimetallbestandteil handelt.

9. Verfahren nach Anspruch 7 oder 8, wobei die Menge des Seltenmetallbestandteils mittels Massenspektroskopie detektiert wird, wobei es sich in einer Ausführungsform bei der Massenspektroskopie um induktiv gekoppelte Massenspektroskopie (ICP-MS) handelt.

10. Verfahren nach einem der Ansprüche 7 bis 9, wobei das Lösen Säurebehandlung und/oder Mikrowellenbehandlung umfasst.

11. Verfahren nach einem der Ansprüche 7 bis 10, wobei es sich bei dem internen Standard um einen isotopangereicherten Seltenmetallbestandteil handelt.

12. Verfahren nach einem der Ansprüche 7 bis 11, wobei zwei Schichten, die eine erste und eine zweite getrocknete Beschichtungszusammensetzung umfassen, in mindestens einem Lösungsmittel gelöst werden, wobei die erste Beschichtungszusammensetzung einen ersten Seltenmetallbestandteil umfasst,
wobei die zweite Beschichtungszusammensetzung einen zweiten Seltenmetallbestandteil umfasst,
und
wobei das mindestens eine Lösungsmittel mindestens einen internen Standard für den ersten Seltenmetallbestandteil und/oder für den zweiten Seltenmetallbestandteil umfasst,
wodurch die absolute und/oder relative Menge der ersten und der zweiten getrockneten Beschichtungszusammensetzung, die in den zwei Schichten enthalten sind, basierend auf der detektierten Menge des ersten und des zweiten Seltenmetallbestandteils bestimmt werden.

13. Beschichtungszusammensetzung zum Bilden einer Schicht, bei der es sich um eine Detektionsschicht oder eine Reaktionsschicht handelt, die in einem Diagnosetestelement enthalten ist, wobei die Beschichtungszusammensetzung einen Seltenmetallbestandteil umfasst, wobei der Seltenmetallbestandteil in anderen Bestandteilen der Beschichtungszusammensetzung nicht in detektierbaren Mengen vorliegt, und wobei der Seltenmetallbestandteil die optischen Eigenschaften der Schicht nicht wesentlich verändert.

14. Diagnosetestelement, das eine Schicht umfasst, bei der es sich um eine Detektionsschicht oder eine Reaktionsschicht handelt, wobei die Schicht getrocknete Beschichtungszusammensetzung umfasst, die einen Seltenmetallbestandteil umfasst, wobei es sich bei dem Seltenmetallbestandteil um ein Metall handelt, das in anderen Bestandteilen der Beschichtungszusammensetzung nicht in detektierbaren Mengen vorliegt, wobei der Seltenmetallbestandteil die optischen Eigenschaften der Schicht nicht wesentlich verändert.

15. Verwendung der Beschichtungszusammensetzung nach Anspruch 13 zum Herstellen eines Diagnosetestelements, das die Beschichtungszusammensetzung in einer Schicht umfasst, bei der es sich um eine Detektionsschicht oder eine Reaktionsschicht handelt.

## Revendications

1. Utilisation d'un composant contenant un métal rare compris dans une composition de revêtement pour la détermination de la quantité de composition de revêtement séchée dans une couche de revêtement, dans laquelle ledit composant contenant un métal rare est une composition comprenant un atome ou ion de métal rare choisi (i) entre V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf et Es ou (ii) entre Li, Rb, Cs et Fr,
dans laquelle ladite couche de revêtement est une couche de détection ou une couche de réaction et
dans laquelle ladite détermination comprend la dissolution de ladite couche de revêtement dans un solvant.

2. Utilisation selon la revendication 1, dans laquelle ledit composant contenant un métal rare est un composant contenant un métal alcalin rare, dans un mode de réalisation dans laquelle ledit composant contenant un métal alcalin rare comprend un ion lithium, rubidium ou césium.

3. Utilisation selon la revendication 1 ou 2, dans laquelle ledit composant contenant un métal rare est un hydroxyde ou sel de métal rare.

4. Utilisation selon l'une quelconque des revendications 1 à 3, dans laquelle ledit composant contenant un métal rare est présent dans la composition de revêtement en une quantité prédéfinie.

5. Utilisation selon l'une quelconque des revendications 1 à 4, dans laquelle ledit composant contenant un métal rare ne réagit pas avec d'autres composants de la composition de revêtement.

6. Utilisation selon l'une quelconque des revendications 1 à 5, dans laquelle ladite couche de détection ou couche de réaction est comprise dans un élément de test de diagnostic.

7. Procédé pour la détermination de la quantité de composition de revêtement séchée dans au moins une couche de revêtement, ledit procédé comprenant :
a) la dissolution de ladite au moins une couche de revêtement comprenant la composition de revêtement séchée dans un solvant, ladite composition de revêtement comprenant un composant contenant un métal rare et ledit solvant comprenant un étalon interne pour ledit composant contenant un métal rare, ledit composant contenant un métal rare étant une composition comprenant un atome ou ion de métal rare choisi (i) entre V, Cr, Mn, Fe, Co, Ni, Cu, Zn, Ga, Ge, As, Zr, Nb, Mo, Tc, Ru, Rh, Pd, Ag, Cd, In, Sn, Sb, Te, La, Hf, Ta, W, Re, Os, Ir, Pt, Au, Hg, Tl, Pb, Bi, Po, Ra, Ac, Rf, Ce, Pr, Nd, Pm, Sm, Eu, Gd, Tb, Dy, Ho, Er, Tm, Yb, Lu, Th, Pa, U, Np, Pu, Am, Cm, Bk, Cf et Es ou (ii) entre Li, Rb, Cs et Fr ;
b) la détection de la quantité dudit composant contenant un métal rare ; et
c) la détermination de la quantité de composition de revêtement séchée comprise dans ladite au moins une couche de revêtement sur la base de la quantité détectée du composant contenant un métal rare ;
dans lequel ladite au moins une couche de revêtement est une couche de détection ou une couche de réaction.

8. Procédé selon la revendication 7, dans lequel ledit composant contenant un métal rare est un composant contenant un métal alcalin rare.

9. Procédé selon la revendication 7 ou 8, dans lequel ladite quantité du composant contenant un métal rare est détectée par spectroscopie de masse, dans un mode de réalisation dans lequel ladite spectroscopie de masse est la spectroscopie de masse à plasma à couplage inductif (ICP-MS).

10. Procédé selon l'une quelconque des revendications 7 à 9, dans lequel ladite dissolution comprend un traitement acide et/ou un traitement par des micro-ondes.

11. Procédé selon l'une quelconque des revendications 7 à 10, dans lequel ledit étalon interne est un composant contenant un métal rare enrichi en isotope.

12. Procédé selon l'une quelconque des revendications 7 à 11, dans lequel deux couches de revêtement comprenant une première composition de revêtement séchée et une seconde composition de revêtement séchée sont dissoutes dans au moins un solvant,
dans lequel la première composition de revêtement comprend un premier composant contenant un métal rare, dans lequel la seconde composition de revêtement comprend un second composant contenant un métal rare et dans lequel ledit au moins un solvant comprend au moins un étalon interne pour ledit premier composant contenant un métal rare et/ou pour ledit second composant contenant un métal rare,
ce qui permet ainsi de déterminer la quantité absolue et/ou relative de ladite première composition de revêtement séchée et de ladite seconde composition de revêtement séchée comprises dans lesdites deux couches de revêtement sur la base de la quantité détectée dudit premier composant contenant un métal rare et dudit second composant contenant un métal rare.

13. Composition de revêtement pour la formation d'une couche de revêtement qui est une couche de détection ou une couche de réaction comprise dans un élément de test de diagnostic, ladite composition de revêtement comprenant un composant contenant un métal rare, ledit métal rare étant un métal qui n'est pas présent en quantités détectables dans d'autres composants de la composition de revêtement et ledit composant contenant un métal rare ne modifiant pas significativement les propriétés optiques de la couche.

14. Élément de test de diagnostic comprenant une couche de revêtement qui est une couche de détection ou une couche de réaction, ladite couche de revêtement comprenant une composition de revêtement séchée comprenant un composant contenant un métal rare, dans lequel ledit métal rare est un métal qui n'est pas présent en quantités détectables dans d'autres composants de la composition de revêtement, dans lequel ledit composant contenant un métal rare ne modifie pas significativement les propriétés optiques de la couche.

15. Utilisation de la composition de revêtement selon la revendication 13 pour la fabrication d'un élément de test de diagnostic comprenant ladite composition de revêtement dans une couche de revêtement qui est une couche de détection ou une couche de réaction.
